(19) Europäisches Patentamt
European Patent Office
Office européen des brevets

(11) Veröffentlichungsnummer : **0 074 651**
**B1**

(12) # EUROPÄISCHE PATENTSCHRIFT

(45) Veröffentlichungstag der Patentschrift : **07.11.84**

(51) Int. Cl.³ : **C 01 B 33/28**, B 01 J 29/28

(21) Anmeldenummer : **82108412.6**

(22) Anmeldetag : **11.09.82**

(54) **Gallium- und/oder indiumhaltige Zeolithe und Verfahren zu deren Herstellung sowie ihre Verwendung.**

(30) Priorität : **16.09.81 DE 3136684**

(43) Veröffentlichungstag der Anmeldung : **23.03.83 Patentblatt 83/12**

(45) Bekanntmachung des Hinweises auf die Patenterteilung : **07.11.84 Patentblatt 84/45**

(84) Benannte Vertragsstaaten : **BE DE FR GB IT NL**

(56) Entgegenhaltungen :
**DE-A- 2 749 024**
**US-A- 2 950 952**
**US-A- 3 431 219**
**US-A- 4 046 826**

(73) Patentinhaber : **HOECHST AKTIENGESELLSCHAFT**
**Postfach 80 03 20**
**D-6230 Frankfurt am Main 80 (DE)**

(72) Erfinder : **Baltes, Herbert, Dr.**
**Johannesallee 24**
**D-6230 Frankfurt am Main 80 (DE)**
Erfinder : **Leupold, Ernst Ingo, Dr.**
**Am Zäunefeld 15**
**D-6392 Neu-Anspach (DE)**
Erfinder : **Litterer, Heinz, Dr.**
**Albert-Schweizer-Allee 39**
**D-6200 Wiesbaden (DE)**
Erfinder : **Wunder, Friedrich, Dr.**
**Jahnstrasse 46**
**D-6093 Flörsheim am Main (DE)**

**EP 0 074 651 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Zeolithe sind kristalline Alumosilikate, bei denen durch eine dreidimensionale Verknüpfung von $SiO_4$- und $AlO_4$-Tetraedern regelmäßige Strukturen mit Hohlräumen und Poren entstehen. Im hydratisierten Zustand sind diese Poren und Hohlräume mit Wasser gefüllt. Dieses läßt sich ohne Beeinflussung der Kristallstruktur entfernen oder durch andere Moleküle ersetzen. Die negativen Ladungen der $AlO_4$-Tetraeder werden durch Kationen kompensiert. Diese können gegen andere positiv geladene Ionen ausgetauscht werden. Die geschilderten Eigenschaften ermöglichen die Verwendung der Zeolithe als Ionenaustauscher, Adsorbentien und Katalysatoren (D. W. Breck : Zeolite Molecular Sieves, 1974).

Zeolithe des X-, Y-, Mordenit, Erionit- und Offretit-Typs beispielsweise besitzen als Katalysatoren für Umwandlungsreaktionen von Kohlenwasserstoffen wie Cracken, Hydrocracken oder Isomerisierungen beträchtliches technisches Interesse. Zeolithe vom Pentasil-Typ (z. B. Zeolith ZSM-5) gewinnen als Katalysatoren für die Umwandlung von Methanol zu Kohlenwasserstoffen steigende Bedeutung.

Aufgrund der zahlreichen Einsatzmöglichkeiten als Katalysatoren besteht großes Interesse an neuen Zeolithen mit spezifischen katalytischen Eigenschaften.

Beispielsweise erhält man sehr interessante Zeolithe, wenn man anstelle von Aluminium oder/und Silicium andere Elemente in das Zeolith-Gerüst einbaut. So wurden unter anderem Zeolithe der Pentasil-Reihe bekannt, die Bor (DE-Offenlegungsschrift 2 830 787), Eisen (DE-Offenlegungsschrift 2 831 611), Arsen (DE-Auslegeschrift 2 830 830), Antimon (DE-Offenlegungsschrift 2 830 787), Vanadin (DE-Offenlegungsschrift 2 831 631), Chrom (DE-Offenlegunsschrift 2 831 630) oder Gallium (BE-Patentschrift 882 484) auf Tetraederplätzen enthalten.

Gegenstand der Erfindung sind gallium- und/oder indiumhaltige Zeolithe, die dadurch gekennzeichnet sind, daß sie

a) die folgende Zusammensetzung besitzen :

$$SiO_2 : (0{,}09 \pm 0{,}07)$$

$$[Al_2O_3 + M_2O_3] : (0{,}12 \pm 0{,}10)$$

$$[Na_2O + K_2O] : (0{,}12 \pm 0{,}10)\ R_2O$$

ausgedrückt in Molverhältnissen von Oxiden, wobei R gleich Cholin $[(CH_3)_3NCH_2CH_2OH]^+$ und M gleich Gallium und/oder Indium ist,
und

b) im Röntgenbeugungsdiagramm die in Tabelle 1 aufgeführten charakteristischen Signale aufweisen :

Tabelle 1

| Netzebenenabstände d [Å] | relative Intensität $I/I_0$ |
|---|---|
| 11,5 ± 0,2 | sehr stark |
| 9,2 ± 0,2 | schwach |
| 7,6 ± 0,2 | schwach bis mittel |
| 6,6 ± 0,1 | mittel bis stark |
| 5,7 ± 0,1 | schwach bis mittel |
| 5,35 ± 0,1 | schwach |
| 4,98 ± 0,1 | schwach |
| 4,56 ± 0,1 | mittel bis stark |
| 4,32 ± 0,1 | stark |
| 4,16 ± 0,1 | schwach |
| 3,81 ± 0,1 | stark bis sehr stark |
| 3,75 ± 0,1 | stark bis sehr stark |
| 3,59 ± 0,1 | stark bis sehr stark |
| 3,30 ± 0,1 | mittel |
| 3,15 ± 0,1 | mittel |
| 2,92 ± 0,1 | schwach |
| 2,86 ± 0,1 | stark bis sehr stark |
| 2,80 ± 0,1 | schwach |
| 2,67 ± 0,1 | schwach |
| 2,49 ± 0,1 | schwach |

Hierbei bedeutet $I_0$ die Intensität des stärksten Signals.
Für die Angabe der Intensitäten in Tabelle 1 gilt :

| relative Intensität | 100 $I/I_0$ |
|---|---|
| sehr stark | 80-100 |
| stark | 50-80 |
| mittel | 20-50 |
| schwach | 0-20 |

Für die erfindungsgemäßen Zeolithe gilt im allgemeinen :

$$Al_2O_3/(Al_2O_3 + M_2O_3) = 0{,}01 - 0{,}99,$$

vorzugsweise

$$Al_2O_3/(Al_2O_3 + M_2O_3) = 0{,}40 - 0{,}99,$$

insbesondere

$$Al_2O_3/(Al_2O_3 + M_2O_3) = 0{,}60 - 0{,}99$$

ausgedrückt in Molverhältnissen der Oxide, wobei M gleich Gallium und/oder Indium ist.

Die erfindungsgemäßen neuen Zeolithe besitzen eine den Zeolithen T (US-Patentsschrift 2 950 952) bzw. ZSM-34 (DE-Offenlegungsschrift 2 749 024) ähnliche Struktur, unterscheiden sich jedoch von diesen in der Zusammensetzung, insbesondere durch den Gallium- bzw. Indiumgehalt.

Die erfindungsgemäßen Zeolithe lassen sich herstellen, indem man Gallium- und/oder Indiumverbindungen mit Aluminium-, Silicium-, Natrium-, Kalium-, Cholinverbindungen und Wasser mischt und in einem geschlossenen Gefäß erhitzt.

Die Ausgangsverbindungen werden im allgemeinen in folgendem Verhältnis eingesetzt, ausgedrückt in Molverhältnissen der Oxide :

$$SiO_2 : (0{,}06 \pm 0{,}058)$$
$$Al_2O_3 : (0{,}06 \pm 0{,}058)$$
$$M_2O_3 : (0{,}2 \pm 0{,}15)$$
$$Na_2O : (0{,}12 \pm 0{,}10)$$
$$K_2O : (0{,}22 \pm 0{,}2)$$
$$R_2O : (50 \pm 40)\ H_2O$$

vorzugsweise im Verhältnis :

$$SiO_2 : (0{,}05 \pm 0{,}04)$$
$$Al_2O_3 : (0{,}05 \pm 0{,}048)$$
$$M_2O_3 : (0{,}2 \pm 0{,}1)$$
$$Na_2O : (0{,}09 \pm 0{,}05)$$
$$K_2O : (0{,}22 \pm 0{,}10)$$
$$R_2O : (20 \pm 10)\ H_2O$$

wobei R gleich Cholin und M gleich Gallium und/oder Indium ist.

Als Verbindungen können beispielsweise eingesetzt werden : Kieselsäuregel, Kaliumsilicat, Natriumsilicat, Aluminiumhydroxid, Aluminiumsulfat, Natriumaluminat, Kaliumaluminat, Aluminiumhalogenide, Aluminiummetahydroxid, Gallium(III)oxid, Gallium(III)nitrat, Gallium(III)sulfat, Gallium(III)halogenide, Gallium(III)hydroxid, Indium(III)oxid, Indium(III)nitrat, Indium(III)sulfat, Indium(III)halogenide, Indium(III)hydroxid, Natriumhydroxid, Natriumsulfat, Natriumhalogenide, Kaliumhydroxid, Kaliumsulfat, Kaliumhalogenide, Cholinhydroxid, Cholinchlorid. Aber auch andere Silicium,- Aluminium-, Gallium-, Indium-, Kalium-, Natrium- und Cholinverbindungen eignen sich für die Herstellung der erfindungsgemäßen Zeolithe.

Das Gemisch der jeweils gewählten Verbindungen mit Wasser wird im allgemeinen 48 bis 2 000 Stunden, vorzugsweise 48 bis 1 000 Stunden lang, auf eine Temperatur zwischen 80 und 160 °C, vorzugsweise zwischen 90 und 150 °C, in einem geschlossenen Gefäß erhitzt.

Die gebildeten Zeolithe werden in üblicher Weise, z. B. durch Filtration, isoliert, gewaschen und getrocknet. Sie können nach bekannten Methoden in die katalytisch aktiven Formen überführt werden, z. B. durch Kalzinierung und/oder Ionenaustausch (D. W. Breck, Zeolite Molecular Sieves, 1974).

Die erfindungsgemäßen Zeolithe zeichnen sich nach ihrer Überführung in die katalytisch aktive Form insbesondere aus durch eine hohe Selektivität und durch eine geringe Koksabscheidung bei der Umwandlung von Methanol in niedrigere Olefine. Diese Reaktion führt man beispielsweise bei Temperaturen von 350 bis 430 °C und einem Wasseranteil im Methanol von 0 bis 80 Gew.-% oder mit Rohmethanol durch.

Die Erfindung soll durch die folgenden Beispiele erläutert werden, wobei die Beispiele aber in keiner Weise einschränkend sein sollen. Alle angegebenen Röntgenbeugungsdaten wurden mit einem computergesteuerten Pulverdiffraktometer D-500 der Firma Siemens aufgenommen. Es wurde Kupfer-K-α-Strahlung verwandt.

Beispiel 1

11,2 g Natriumaluminat (54 Gew.-% $Al_2O_3$, 41 Gew.-% $Na_2O$), 11,15 g Galliumtrioxid 5,9 g Natriumhydroxid, 5,3 g Kaliumhydroxid, und 45,6 g Cholinchlorid werden in 150 g Wasser gelöst. In Diese Lösung werden 117 g 40 gew.-%iges kolloidales Kieselgel eingebracht. Die entstandene Mischung wird homogenisiert und 8 Tage im geschlossenen Gefäß auf 150 °C erhitzt. Das entstandene Produkt wird abfiltriert, mit Wasser gewaschen und bei 120 °C getrocknet.

Die chemische Analyse ergibt folgende Zusammensetzung, ausgedrückt in Molverhältnissen von Oxiden :

$$SiO_2 : 0{,}098$$
$$Al_2O_3 : 0{,}031$$
$$Ga_2O_3 : 0{,}050$$
$$Na_2O : 0{,}046$$
$$K_2O : 0{,}051\ R_2O,$$

wobei R = Cholin ist.

Das Ergebnis der Röntgenbeugungsanalyse ist in Tabelle 2 wiedergegeben.

Tabelle 2

| Netzebenenabstände d [Å] | relative Intensität 100 $I/I_0$ |
|---|---|
| 11,50 | 100 |
| 9,16 | 5 |
| 7,58 | 18 |
| 6,62 | 49 |
| 6,33 | 8 |
| 5,74 | 16 |
| 5,35 | 3 |
| 4,98 | 2 |
| 4,56 | 37 |
| 4,33 | 60 |
| 4,16 | 6 |
| 3,81 | 92 |
| 3,76 | 91 |
| 3,59 | 80 |
| 3,30 | 33 |
| 3,16 | 41 |
| 2,92 | 10 |
| 2,85 | 88 |
| 2,80 | 6 |
| 2,67 | 7 |
| 2,49 | 8 |

Beispiel 2

2,23 g Galliumtrioxid, 7,41 g Aluminiumhydroxid, 10,6 g Natriumhydroxid, 5,30 g Kaliumhydroxid und 45,6 g Cholinchlorid werden in 150 g Wasser gelöst. In diese Lösung werden 117 g 40 gew.-%iges kolloidales Kieselgel eingebracht. Die entstandene Mischung wird homogenisiert und 720 h im

geschlossenen Gefäß auf 105 °C erhitzt. Nach Aufarbeitung wie in Beispiel 1 erhält man ein kristallines Produkt der folgenden Zusammensetzung, ausgedrückt in Molverhältnissen von Oxiden :

$$SiO_2 : 0{,}071$$
$$Al_2O_3 : 0{,}014$$
$$Ga_2O_3 : 0{,}040$$
$$Na_2O : 0{,}031$$
$$K_2O : 0{,}020\ R_2O,$$

wobei R = Cholin ist.

Die Röntgendaten entsprechen den in Tabelle 1 angegebenen.

Beispiel 3

Wie in Beispiel 2 wird eine Mischung hergestellt aus 5,6 g Natriumaluminat, 11,0 g Galliumtrioxid, 7,2 g Natriumhydroxid, 5,3 g Kaliumhydroxid, 45,6 g Cholinchlorid, 117 g 40 gew.-%igem kolloidalem Kieselgel und 150 g Wasser. Diese Mischung wird 1 200 h im geschlossenen Gefäß auf 100 °C erhitzt. Nach Aufarbeitung wie in Beispiel 1 erhält man ein kristallines Produkt mit der folgenden Zusammensetzung, ausgedrückt in Molverhältnissen von Oxiden :

$$SiO_2 : 0{,}040$$
$$Al_2O_3 : 0{,}044$$
$$Ga_2O_3 : 0{,}024$$
$$Na_2O : 0{,}033$$
$$K_2O : 0{,}051\ R_2O,$$

wobei R = Cholin ist.

Das Produkt zeigt die in Tabelle 1 angegebenen Röntgensignale.

Beispiel 4

11,2 g Natriumaluminat (54 Gew.-% $Al_2O_3$, 41 Gew.-% $Na_2O$), 5,28 g Indiumtrichlorid, 5,9 g Natriumhydroxid 5,3 g Kaliumhydroxid, und 45,6 Cholinchlorid werden in 150 g Wasser gelöst. In diese Lösung werden 117 g 40 gew.-%iges kolloidales Kieselgel eingebracht. Die entstandene Mischung wird homogenisiert und 8 Tage im geschlossenen Gefäß auf 150 °C erhitzt. Das entstandene Produkt wird abfiltriert, mit Wasser gewaschen und bei 120 °C getrocknet.

Die chemische Analyse ergibt folgende Zusammensetzung, ausgedrückt in Molverhältnissen von Oxiden :

$$SiO_2 : 0{,}078$$
$$Al_2O_3 : 0{,}008$$
$$In_2O_3 : 0{,}031$$
$$Na_2O : 0{,}032$$
$$K_2O : 0{,}038\ R_2O,$$

wobei R = Cholin ist.

Das Produkt zeigt die in Tabelle 1 angegebenen Röntgensignale.

**Ansprüche**

1. Gallium- und/oder indiumhaltige Zeolithe, dadurch gekennzeichnet, daß sie
   a) die folgende Zusammensetzung besitzen :

$$SiO_2 : (0{,}09 \pm 0{,}07)$$

$$[Al_2O_3 + M_2O_3] : (0{,}12 \pm 0{,}10)$$

$$[Na_2O + K_2O] : (0{,}12 \pm 0{,}10)\ R_2O$$

ausgedrückt in Molverhältnissen von Oxiden, wobei R gleich Cholin und M gleich Gallium und/oder Indium ist,
und

b) im Röntgenbeugungsdiagramm die in Tabelle 1 aufgeführten charakteristischen Signale aufweisen :

Tabelle 1

| Netzebenenabstände d [Å] | relative Intensität $I/I_0$ |
|---|---|
| 11,5 ± 0,2 | sehr stark |
| 9,2 ± 0,2 | schwach |
| 7,6 ± 0,2 | schwach bis mittel |
| 6,6 ± 0,1 | mittel bis stark |
| 5,7 ± 0,1 | schwach bis mittel |
| 5,35 ± 0,1 | schwach |
| 4,98 ± 0,1 | schwach |
| 4,56 ± 0,1 | mittel bis stark |
| 4,32 ± 0,1 | stark |
| 4,16 ± 0,1 | schwach |
| 3,81 ± 0,1 | stark bis sehr stark |
| 3,75 ± 0,1 | stark bis sehr stark |
| 3,59 ± 0,1 | stark bis sehr stark |
| 3,30 ± 0,1 | mittel |
| 3,15 ± 0,1 | mittel |
| 2,92 ± 0,1 | schwach |
| 2,86 ± 0,1 | stark bis sehr stark |
| 2,80 ± 0,1 | schwach |
| 2,67 ± 0,1 | schwach |
| 2,49 ± 0,1 | schwach |

Hierbei bedeutet $I_0$ die Intensität des stärksten Signals.

2. Gallium- und/oder indiumhaltige Zeolithe nach Anspruch 1, dadurch gekennzeichnet, daß gilt :

$$Al_2O_3/(Al_2O_3 + M_2O_3) = 0{,}01 - 0{,}99,$$

ausgedrückt in Molverhältnissen der Oxide, wobei M gleich Gallium und/oder Indium ist.

3. Gallium- und/oder indiumhaltige Zeolithe nach Anspruch 1 oder 2, dadurch gekennzeichnet, daß gilt :

$$Al_2O_3/(Al_2O_3 + M_2O_3) = 0{,}40 - 0{,}99,$$

ausgedrückt in Molverhältnissen der Oxide, wobei M gleich Gallium und/oder Indium ist.

4. Gallium- und/oder indiumhaltige Zeolithe nach einem der Ansprüche 1 bis 3, dadurch gekennzeichnet, daß gilt :

$$Al_2O_3/(Al_2O_3 + M_2O_3) = 0{,}60 - 0{,}99,$$

ausgedrückt in Molverhältnissen der Oxide, wobei M gleich Gallium und/oder Indium ist.

5. Verfahren zur Herstellung von gallium- und/oder indiumhaltigen Zeolithen nach einem der Ansprüche 1 bis 4, dadurch gekennzeichnet, daß man eine Mischung aus Silicium-, Aluminium-, Natrium-, Kalium- und Cholinverbindungen, Wasser sowie Gallium- und/oder Indiumverbindungen herstellt, die folgende Zusammensetzung hat, ausgedrückt in Molverhältnissen der Oxide :

$$SiO_2 : (0{,}06 \pm 0{,}058)$$
$$Al_2O_3 : (0{,}06 \pm 0{,}058)$$
$$M_2O_3 : (0{,}2 \pm 0{,}15)$$
$$Na_2O : (0{,}12 \pm 0{,}10)$$
$$K_2O : (0{,}22 \pm 0{,}2)$$
$$R_2O : (50 \pm 40)\ H_2O$$

wobei R gleich Cholin und M gleich Gallium und/oder Indium ist, und diese Mischung in einem geschlossenen Gefäß erhitzt.

6. Verfahren nach Anspruch 5, dadurch gekennzeichnet, daß die zu erhitzende Mischung folgende Zusammensetzung hat, ausgedrückt in Molverhältnissen der Oxide :

$$SiO_2 : (0{,}05 \pm 0{,}04)$$
$$Al_2O_3 : (0{,}05 \pm 0{,}048)$$
$$M_2O_3 : (0{,}2 \pm 0{,}1)$$

$Na_2O$ : (0,09 ± 0,05)
$K_2O$ : (0,22 ± 0,10)
$R_2O$ : (20 ± 10) $H_2O$

wobei R gleich Cholin und M gleich Gallium und/oder Indium ist.

7. Verwendung von gallium- und/oder indiumhaltigen Zeolithen nach einem der Ansprüche 1 bis 4 als Katalysatoren bei der Herstellung von $C_2$ bis $C_4$-Olefinen aus Methanol.

**Claims**

1. A gallium-containing and/or indium-containing zeolite which

a) has the following composition :

$$SiO_2 : (0.09 \pm 0.07)$$

$$[Al_2O_3 + M_2O_3] : (0.12 \pm 0.010)$$

$$[Na_2O + K_2O] : (0.12 \pm 0.010)\ R_2O$$

expressed as molar ratios of oxides, wherein R is choline and M is gallium and/or indium, and

b) exhibits, in the X-ray diffraction diagram, the characteristic signals listed in Table 1 :

Table 1

| Interlayer spacing d [Å] | Relative intensity $I/I_0$ |
|---|---|
| 11.5 ± 0.2 | very strong |
| 9.2 ± 0.2 | weak |
| 7.6 ± 0.2 | weak to medium |
| 6.6 ± 0.1 | medium to strong |
| 5.7 ± 0.1 | weak to medium |
| 5.35 ± 0.1 | weak |
| 4.98 ± 0.1 | weak |
| 4.56 ± 0.1 | medium to strong |
| 4.32 ± 0.1 | strong |
| 4.16 ± 0.1 | weak |
| 3.81 ± 0.1 | strong to very strong |
| 3.75 ± 0.1 | strong to very strong |
| 3.59 ± 0.1 | strong to very strong |
| 3.30 ± 0.1 | medium |
| 3.15 ± 0.1 | medium |
| 2.92 ± 0.1 | weak |
| 2.86 ± 0.1 | strong to very strong |
| 2.80 ± 0.1 | weak |
| 2.67 ± 0.1 | weak |
| 2.49 ± 0.1 | weak |

In the above table, $I_0$ denotes the intensity of the strongest signal.

2. A gallium-containing and/or indium-containing zeolite as claimed in claim 1, wherein the following applies :

$$Al_2O_3/(Al_2O_3 + M_2O_3) = 0.01 - 0.99,$$

expressed as molar ratios of the oxides, wherein M is gallium and/or indium.

3. A gallium-containing and/or indium-containing zeolite as claimed in claim 1 or 2, wherein the following applies :

$$Al_2O_3/(Al_2O_3 + M_2O_3) = 0.40 - 0.99,$$

expressed as molar ratios of the oxides, wherein M is gallium and/or indium.

4. A gallium-containing and/or indium-containing zeolite as claimed in any one of claims 1 to 3, wherein the following applies :

$$Al_2O_3/(Al_2O_3 + M_2O_3) = 0.60 - 0.99,$$

expressed as molar ratios of the oxides, wherein M is gallium and/or indium.

5. A process for the preparation of a gallium-containing and/or indium-containing zeolite as claimed in any one of claims 1 to 4, wherein a mixture of silicon compounds, aluminum compounds, sodium compounds, potassium compounds and choline compounds, water and gallium compounds and/or indium compounds is prepared, the mixture having the following composition, expressed as molar ratios of the oxides :

$$SiO_2 : (0.06 \pm 0.058)$$
$$Al_2O_3 : (0.06 \pm 0.058)$$
$$M_2O_3 : (0.2 \pm 0.15)$$
$$Na_2O : (0.12 \pm 0.10)$$
$$K_2O : (0.22 \pm 0.2)$$
$$R_2O : (50 \pm 40)\ H_2O$$

wherein R is choline and M is gallium and/or indium, and this mixture is heated in a closed vessel.

6. A process as claimed in claim 5, wherein the mixture to be heated has the following composition, expressed as molar ratios of the oxides :

$$SiO_2 : (0.05 \pm 0.04)$$
$$Al_2O_3 : (0.05 \pm 0.048)$$
$$M_2O_3 : (0.2 \pm 0.1)$$
$$Na_2O : (0.09 \pm 0.05)$$
$$K_2O : (0.22 \pm 0.10)$$
$$R_2O : (20 \pm 10)\ H_2O$$

wherein R is choline and M is gallium and/or indium.

7. Use, as catalysts, in the production of $C_2$-$C_4$-olefins from methanol, of gallium-containing and/or indium-containing zeolites as claimed in any one of claims 1 to 4.

**Revendications**

1. Zéolites contenant du gallium et/ou de l'indium, caractérisées en ce que :
a) elles ont la composition suivante :

$$SiO_2 : (0.09 \pm 0,07)$$

$$[Al_2O_3 + M_2O_3] : (0,12 \pm 0,10)$$

$$[Na_2O + K_2O] : (0,12 \pm 0,10)\ R_2O,$$

exprimée en rapports molaires d'oxydes, le symbole R désignant la choline et le symbole M le gallium et/ou l'indium,
et
b) leur diagramme de diffraction des rayons X présente les signaux caractéristiques qui sont indiqués dans le tableau 1 :

(Voir Tableau 1, p. 9)

Tableau 1

| Distance entre plans réticulaires d [Å] | Intensité relative $I/I_0$ |
|---|---|
| 11,5 ± 0,2 | très forte |
| 9,2 ± 0,2 | faible |
| 7,6 ± 0,2 | faible à moyenne |
| 6,6 ± 0,1 | moyenne à forte |
| 5,7 ± 0,1 | faible à moyenne |
| 5,35 ± 0,1 | faible |
| 4,98 ± 0,1 | faible |
| 4,56 ± 0,1 | moyenne à forte |
| 4,32 ± 0,1 | forte |
| 4,16 ± 0,1 | faible |
| 3,81 ± 0,1 | forte à très forte |
| 3,75 ± 0,1 | forte à très forte |
| 3,59 ± 0,1 | forte à très forte |
| 3,30 ± 0,1 | moyenne |
| 3,15 ± 0,1 | moyenne |
| 2,92 ± 0,1 | faible |
| 2,86 ± 0,1 | forte à très forte |
| 2,80 ± 0,1 | faible |
| 2,67 ± 0,1 | faible |
| 2,49 ± 0,1 | faible |

$I_0$ désignant l'intensité du signal le plus fort.

2. Zéolites contenant du gallium et/ou de l'indium selon la revendication 1, caractérisées par la relation suivante :

$$Al_2O_3/(Al_2O_3 + M_2O_3) = 0{,}01 - 0{,}99,$$

exprimée en rapports molaires des oxydes, le symbole M désignant le gallium et/ou l'indium.

3. Zéolites contenant du gallium et/ou de l'indium selon l'une des revendications 1 et 2, caractérisées par la relation :

$$Al_2O_3/(Al_2O_3 + M_2O_3) = 0{,}40 - 0{,}99,$$

exprimée en rapports molaires des oxydes, le symbole M représentant le gallium et/ou l'indium.

4. Zéolites contenant du gallium et/ou de l'indium selon l'une quelconque des revendications 1 à 3, caractérisées par la relation suivante :

$$Al_2O_3/(Al_2O_3 + M_2O_3) = 0{,}60 - 0{,}99,$$

exprimée en rapports molaires des oxydes, le symbole M représentant le gallium et/ou l'indium.

5. Procédé de préparation de zéolites contenant du gallium et/ou de l'indium selon l'une quelconque des revendications 1 à 4, procédé caractérisé en ce qu'on prépare, à partir d'un mélange constitué de composés du silicium, de l'aluminium, du sodium, du potassium et de la choline, d'eau et de composés du gallium et/ou de l'indium, un mélange qui a la composition suivante (exprimée en rapports molaires des oxydes) :

$$\begin{aligned} SiO_2 &: (0{,}06 \pm 0{,}058) \\ Al_2O_3 &: (0{,}06 \pm 0{,}058) \\ M_2O_3 &: (0{,}2 \pm 0{,}15) \\ Na_2O &: (0{,}12 \pm 0{,}10) \\ K_2O &: (0{,}22 \pm 0{,}2) \\ R_2O &: (50 \pm 40)\ H_2O, \end{aligned}$$

le symbole R représentant la choline et le symbole M le gallium et/ou l'indium, et on chauffe ce mélange dans un récipient fermé.

6. Procédé selon la revendication 5, caractérisé en ce que le mélange à chauffer a la composition suivante (exprimée en rapports molaires des oxydes) :

$SiO_2$ : (0,05 ± 0,04)
$Al_2O_3$ : (0,05 ± 0,048)
$M_2O_3$ : (0,2 ± 0,1)
$Na_2O$ : (0,09 ± 0,05)
$K_2O$ : (0,22 ± 0,10)
$R_2O$ : (20 ± 10) $H_2O$,

le symbole R représentant la choline et le symbole M le gallium et/ou l'indium.

7. Application de zéolites contenant du gallium et/ou de l'indium selon l'une quelconque des revendications 1 à 4, comme catalyseurs dans la préparation d'oléfines en $C_2$ à $C_4$ à partir de méthanol.